# EUROPEAN PATENT APPLICATION

(11) **EP 3 493 004 A1**
(43) Date of publication of application: **05.06.2019**
(21) Application number: 17425122.3
(22) Date of filing: 01.12.2017
(51) Int. Cl.: G05B 19/05, G01N 33/04

(54) **PLANT**

(71) Applicant: Zappala´ SPA, 95019 Zafferana Etnea (CT) (IT)
(72) Inventor: Zappala', Luigi, 95019 Zafferana Etnea (CT) (IT); Salemi, Sebastiano, 95019 Zafferana Etnea (CT) (IT)

(57) **Abstract**

Automatic vision on-line system with cameras for detection of foreign bodies on continuous flow of filled pasta filata mixtures, milk derivatives and other food products.

## Description

The quality of food inevitably also comes from their safety. In addition, the recognition and elimination of foreign-based products are now recognized as a key requirement to meet GDO's demands, which require its suppliers to adopt private product-certification volunteers, such as BRC and IFS. To meet these needs, technologies are increasingly being designed to identify foreign bodies in any type of food and drink.

### The management of foreign bodies

For a foreign body is meant a solid material, which does not belong by composition or reasonably expected of the product in which it is detected. It can be a bone fragment in an animal food or a stem in a plant origin, or alien to the food itself, such as an insect or a piece of metal with which the product has been contaminated along the supply chain. They are foreign bodies such as metals, glass, ceramics, stones, bones or lichens, rubber, wood, various materials and even those of a similar nature to the product, such as shells, hazelnuts, seeds. The assumption and implementation of a self-control plan cannot ignore the detection of foreign bodies, thanks to the adoption of efficient inspection procedures. The origin of contamination by foreign bodies can occur throughout the entire production chain, and that is why a good strategy is to monitor all potential sources of origin, from raw materials to the environment, from tools to machinery. And so, along with the application of some risk-reducing behavioral norms, such as regular verification of the integrity of hazardous materials, the elimination of what may be a hazard and the use of more easily identifiable materials, the adoption of monitoring systems on both raw materials and finished products is crucial. The company must be able to identify the causes and take any corrective action to prevent or reduce the risk, to demonstrate to the control bodies that they have done everything possible to prevent contamination from foreign bodies.

### The main technologies used

The cases complained of by Rasff (the European Rapid Alert System for Food and Feed), contamination by foreign bodies are rather sporadic, but they exist and are therefore not a risk to be underestimated. In the last few years, in the EU, there have been cases of bone-waste chocolate, semi-skimmed UHT milk with plastic bits, canned tuna with bones of animals, pieces of glass in pizza and metal pieces in a smoked salmon. A real case is missing for those foods produced and sold only in Italy, as there is no monitoring by the Ministry of Health. Food companies work side-by-side with visual inspection (for example, in pasta factories, they tend to wear colored gloves instead of light ones to make visible the presence in the dough) to physical barriers that physically block the passage of bodies strangers, perhaps with the use of sieves, which can also aid their rapid removal, and detection systems such as metal detectors, X-rays. The metal detector only detects metallic contaminants. It is not therefore a sufficient tool because splinters of glass or wood, small stones or foreign plastic bodies can be contained in the final product, and escape the analysis. So, X-ray detection is one of the most widely used in the food industry. Since the 1990s, the food industry uses imaging machines that utilize X-ray technology that, thanks to their wavelength, can penetrate materials that are opaque to visible light. The transparency of an X-ray material depends on its density: the denser the material is, the less it will be crossed by the X-ray. The foreign bodies are usually dense materials such as glass, bones and metals and are well visible in scans. The inspection machine is accompanied by a transport system that can discard any non-compliant product. X-rays do not compromise in any way affecting the safety or nutritional value of processed foods. Inspectors work by combining X-rays with image processing technologies. The X-ray beam passes through the product and the conveyor belt and strikes a line sensor that converts the radiation transmitted to electronic signals. The processing system generates images that, through processing software, are analyzed to detect possible contamination or defects in the product.

### Limitations of current inspection systems (x-ray and metal-detector)

There are some polluting bodies that cannot be identified by current pollutant foreign polluting systems: plastic and plastic packaging, wood, teflon, particles of unrefined organic substances, carbonaceous aggregates of high temperature proteins, hairs, fragments of paper, insects and fragments of insects, etc ...

### OBJECTIVE OF INVESTMENT

Eliminate or reduce the risks of contamination of foreign bodies in milk cheeses and cheeses (within the pasta / cheese structure) with innovative automatic non-invasive and non-destructive inspection and monitoring systems.

The present invention serves to overcome the current limitations of X-ray and metal-detector systems against some foreign bodies (for this purpose) it is necessary to apply other technological systems that allow identification of bodies or particles not detectable by current inspection systems.

### DESCRIPTION OF THE PLAN

The present invention serves to fill in the impossibility of detecting particulate matter and foreign pollutants as already highlighted.

This is an innovative automatic system with high resolution specialty cameras and software that allow on-line inspection and control on a still hot extruded cheese stream (about 65-70 ° C) before being sent to forming.

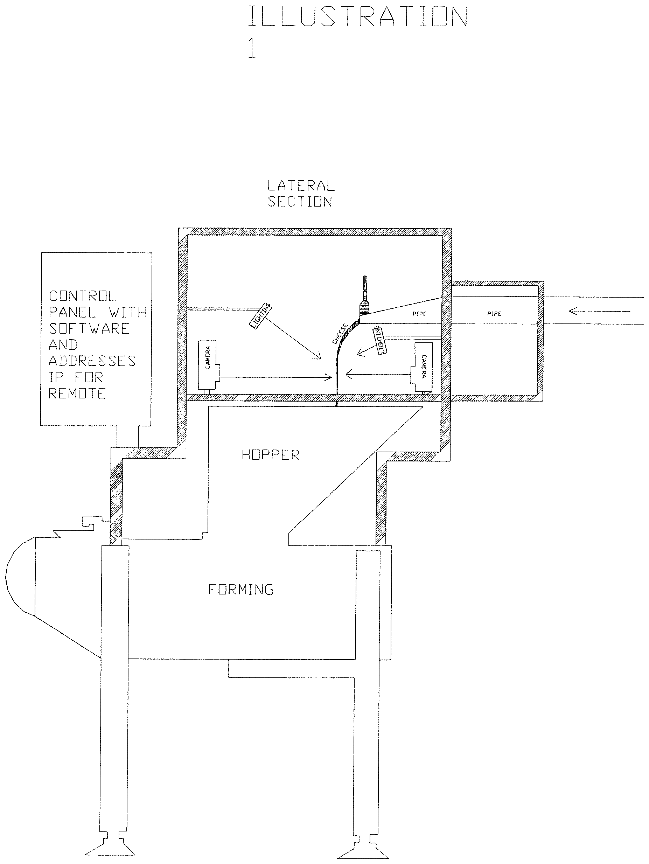

In the specific case of yarn-type cheeses, the cheese's plastic and extensible capacity is used when it is at temperatures above 60 ° C; Under these conditions, the spun cheese can be extruded and extended with special equipment until a continuous thin sheet of a few millimeters thick (about 4-5 mm.) and about 30 cm wide. On this stream of rolled cheese, in a specific cascade fall, the control and monitoring system is applied using high resolution special cameras aimed at the surface of the cheese in order to detect any different color and density of the cheese which is checked at that time. (SEE FIG. 1)

The plant consists of:
- a supporting structure (frame) that is fixed to any screw conveyor / molding machine and swivel drum mold.
- Extrusion tube for rolling a thin layer of continuous cheese, equipped with a pneumatic door to open or close the passage of the hot cheese flow.
- No. 2 special high-resolution cameras for continuous viewing of cheese flow.
- No. 2 lighting fixtures.
- Closing / opening plate with air piston for separating and expelling the cheese to be discarded.
- No. 1 electrical cabinet with dedicated PLC for program management and all sensors.
- Software that manages a specific program for the recognition of foreign particles of different shape, color and density compared to cheese to be controlled.
- Program that manages remote on-line connections for viewing and control through specific IP addresses

## Claims

1. Online auto vision system with high resolution special cameras for the control and monitoring of foreign bodies on a continuous flow of yogurt pasta and milk derivatives and other food products.

2. The plant is **characterized by**: a supporting structure (frame) which is fixed to any screw conveyor / molding machine and rotary drum mold; an extrusion tube for rolling a thin layer of continuous cheese, provided with a pneumatic door to open or close the passage of the hot cheese flow; n ° 2 special cameras for continuous viewing of cheese flow; n ° 2 lighting fixtures; Closing / opening plate with air piston for the separation and expulsion of the cheese to be discarded; n ° 1 electrical cabinet with dedicated PLC for program management and all sensors.

3. The plant is **characterized by** the detection of foreign bodies that are not currently detected by "machines / equipment with magnetic detection system" and "machines / equipment with X-Ray detection system" for cheeses yarn, pasta, milk and other food products.

4. The plant is **characterized by** a special software that manages a specific program for the recognition of foreign particles of different shape, color and density compared to cheese and / or foodstuffs to be controlled.

5. The system is **characterized by** a program that manages remote on-line connections for viewing and monitoring via specific "IP" addresses for remote connection
